**Europäisches Patentamt**

**European Patent Office**

**Office européen des brevets**

(19)

(11) Veröffentlichungsnummer: **0 378 139**
**A2**

(12) # EUROPÄISCHE PATENTANMELDUNG

(21) Anmeldenummer: **90100276.6**

(22) Anmeldetag: **08.01.90**

(51) Int. Cl.5: **C12Q 1/56, G01N 33/86**

(30) Priorität: **10.01.89 DE 3900493**

(43) Veröffentlichungstag der Anmeldung:
**18.07.90 Patentblatt 90/29**

(84) Benannte Vertragsstaaten:
**AT BE CH DE DK ES FR GB GR IT LI LU NL SE**

(71) Anmelder: **Dr. Karl Thomae GmbH**
**Postfach 1755**
**D-7950 Biberach 1(DE)**

(72) Erfinder: **Krause, Jürgen, Dr. Dipl.-Chem.**
**Freiherr von Schadstrasse 17**
**D-7951 Warthausen(DE)**
Erfinder: **Haarmann, Walter, Dr.**
**Schlierholzstrasse 27**
**D-7950 Biberach 1(DE)**

(54) **Verfahren zur Bestimmung der fibrinolytischen Gesamtaktivität im Plasma.**

(57) Die vorliegende Anmeldung betrifft ein Verfahren zur Bestimmung der fibrinolytischen Gesamtaktivität im Plasma, welches, dadurch gekennzeichnet, daß man einer verdünnten plättchenarmen Plasmaprobe

a) eine zur Trübungsbildung ausreichende Menge an Fibrin zusetzt oder diese in situ erzeugt und die zeitabhängige Trübungsänderung oder die zeitabhängige Bildung von Fibrinspaltprodukten mißt oder

b) ein chromogenes Plasminsubstrat und eine nicht zur Trübungsbildung ausreichende Menge an Fibrin, Fibrinogenspaltprodukten oder eines Fibrin in situ erzeugenden Enzymes zusetzt und die zeitabhängige Farbbildung mißt.

EP 0 378 139 A2

## Verfahren zur Bestimmung der fibrinolytischen Gesamtaktivität im Plasma

In der DE-A-3.502.878 wird bereits ein Verfahren zur Bestimmung des fibrinolytischen Zustandes von Plasma beschrieben, welches dadurch gekennzeichnet ist, daß man einer nativen Plasmaprobe

a) eine zur Trübungsbildung ausreichende Menge an Fibrin zusetzt oder diese in situ erzeugt und die Trübung oder die gebildeten Fibrinspaltprodukte mißt oder

b) ein chromogenes Plasminsubstrat und eine nicht zur Trübungsbildung ausreichende Menge an Fibrin, Fibrinogenspaltprodukten oder eines Fibrin in situ erzeugenden Enzymes zusetzt und die gebildete Farbe mißt.

Durch das vorstehend gekennzeichnete Verfahren soll eine Bestimmung der hyperfibrinolytischen Aktivität des Plasmas in "relativ kurzer Zeit" ermöglicht werden, was insbesondere für die Verfolgung einer Fibrinolysetherapie von Bedeutung wäre. Hierzu wird zweckmäßigerweise eine bestimmte Menge an Plasminogenaktivator zu dem zu untersuchenden Plasma zugesetzt, wobei bevorzugt therapeutisch angewandte Konzentrationen an Plasminogenaktivatoren eingesetzt werden. Bei dieser Ausführungsform wird also die mögliche Reaktivität des Plasmas in Gegenwart von definierten Mengen Plasminogenaktivator bestimmt. Hierdurch kann man feststellen, wie sich der fibrino lytische Zustand des Plasmas ändert, wenn die, für eine Fibrinolysetherapie vorgesehene Therapeutika, z.B. t-PA, Urokinase, Prourokinase, Streptokinase oder eines ihrer Derivate, zugesetzt werden.

In der Praxis hat es sich jedoch gezeigt, daß die vorstehend beschriebene Bestimmungsmethode für die akute Überwachung einer Lysetherapie, insbesondere mit niederen Konzentrationen eines Plasminogenaktivators wie t-PA, z.B. mit einem rt-PA-Gehalt zwischen 2 ng/ml und 1000 ng/ml, vorzugsweise zwischen 2 ng/ml und 100 ng/ml, viel zu zeitaufwendig und nicht ausreichend empfindlich ist.

Der vorliegenden Erfindung liegt daher die Aufgabe zugrunde, einen Fibrinolyseglobaltest, der sich automatisieren läßt und photometrisch ausgewertet werden kann, zu schaffen, welcher die Nachteile der oben erwähnten Methode vermeidet und insbesondere in kurzer Zeit Ergebnisse liefert, welche den in vivo Zustand zuverlässig widerspiegeln.

Erfindungsgemäß wird diese Aufgabe durch die Verwendung von verdünntem plättchenarmem Plasma (PPP = platelet poor plasma) an Stelle von nativem Plasma gelöst.

Das erfindungsgemäße Verfahren zur Bestimmung der fibrinolytischen Gesamtaktivität im Plasma ist somit dadurch gekennzeichnet, daß man einer verdünnten plättchenarmen Plasmaprobe

a) eine zur Trübungsbildung ausreichende Menge an Fibrin zusetzt oder diese in situ erzeugt und die zeitabhängige Trübungsänderung oder die zeitabhängige Bildung von Fibrinspaltprodukten mißt oder

b) ein chromogenes Plasminsubstrat und eine nicht zur Trübungsbildung ausreichende Menge an Fibrin, Fibrinogenspaltprodukten oder eines Fibrin in situ erzeugenden Enzymes zusetzt und die zeitabhängige Farbbildung mißt.

Unter Trübungsmessung wird hierbei im Rahmen der Erfindung die Messung der Trübungszunahme und/oder -abnahme pro Zeiteinheit, die Messung der Zeit bis zum Erreichen des Trübungsmaximus oder die Messung der Zeit bis zum Erreichen einer bestimnten Trübungsabnahme oder -zunahme verstanden, jeweils bezogen auf das Trübungsmaximum. Auch eine Kombination dieser Meßmöglichkeiten kann angewendet werden.

Die Farbmessung kann durch kinetische Verfolgung der Farbbildung erfolgen, wobei die Extinktionsänderung pro Zeiteinheit ermittelt wird. Ebenso kann die nach einer vorgegebenen Zeit erreichte Extinktion bestimmt werden oder es kann die Farbbildungsreaktion nach einer vorgegebenen Zeit abgestoppt und zu beliebiger Zeit später dann die gebildete Farbe gemessen werden.

Die Messung der gebildeten Fibrinspaltprodukte kann mit handelsüblichen Testreagenzien bzw. -verfahren erfolgen. Beispiele sind Staphylokokken-clumping-Test (Hersteller: Boehringer Mannheim GmbH) oder immunologische Verfahren z.B. über antikörperbeschichtete Latexpartikel (Hersteller: Wellcome Corp.).

Das Abstoppen der Farbbildungsreaktion kann durch Zusatz geeigneter Inhibitoren, vorzugsweise Plasmininhibitoren oder Säuren wie z.B. Essigsäure oder Zitronensäure, erfolgen. Bevorzugt wird die Zeit bis zum Erreichen einer bestimmten Extinktion gemessen.

Das vorstehend definierte erfindungsgemäße Verfahren ergibt eine Bestimmung der hyperfibrinolytischen Aktivität des Plasmas. Dabei lösen bereits im Plasma vorhandene oder entstandene Plasminogenaktivatoren die Reaktion aus. Diese Ausführungsform des erfindungsgemäßen Verfahrens eignet sich besonders für die Verfolgung einer Fibrinolysetherapie. Man kann damit feststellen, wie sich der fibrinolytische Zustand des nativen Plasmas im Verlauf der Therapie ändert.

Gemäß einer weiteren Ausführungsform des erfindungsgemäßen Verfahrens wird außerdem eine bestimmte Menge an Plasminogenaktivator dem Blut oder Plasma zugesetzt, wobei bevorzugt therapeutisch angewandte Konzentrationen eingesetzt werden. Bei dieser Ausführungsform des Verfahrens bestimmt man die mögliche Reaktivität des Plasmas in Gegenwart von definierten Mengen Plasminogenaktivator. Dies ist besonders wichtig zum Erstellen einer Eichkurve und für die Einleitung einer Fibrinolysetherapie. Man kann dabei feststellen, wie sich der fibrinolytische Zustand des Plasmas ändert, wenn die für eine Fibrinolysetherapie vorgesehenen Therapeutika, z.B. t-PA, Urokinase, Prourokinase, Streptokinase oder eines ihrer Derivate, zugesetzt werden.

Gemäß einer bevorzugten Ausführungsform wird das Fibrin in situ durch Zusatz von Thrombin oder einem thrombin-ähnlichen Enzym wie Batroxobin oder Arvin erzeugt.

Als Fibrinogenspaltprodukte werden bevorzugt solche verwendet, die durch Behandlung von Fibrinogen mit Bromcyan erhalten werden (I. H. Verheijen, Thromb. Haemostas 48, 266-269 (1982)). Bevorzugt werden Konzentrationen von 10 bis 150 µg/ml eingesetzt.

Fibrinmonomere werden hergestellt durch Behandlung von Fibrinogen mit Batroxobin, Inaktivierung des Batroxobins mit Diisopropylfluorphosphat, Entfernen und anschließendem Auflösen des Fibrinolats in 1- bis 3-molarer Harnstofflösung. Bevorzugt werden Konzentrationen von 1 bis 100 µg/ml Fibrinmonomer eingesetzt.

Als chromogenes Plasminsubstrat kann jedes Plasminsubstrat verwendet werden, aus welchem unter der Einwirkung von Plasmin eine zur Farbbildung geeignete Gruppe abgespalten wird. Als farbbildende Gruppen kommen hierbei sowohl solche in Frage, die direkt photometrisch gemessen werden können wie z.B. Nitroanilin, Dinitroanilin und deren Derivate als auch solche Verbindungen, die durch Reaktion mit einer weiteren Komponente eine Farbbildung bewirken.

Bei der Ausführungsform des erfindungsgemäßen Verfahrens unter Zusatz eines Plasminogenaktivators kann man als Plasminogenaktivator beispielsweise t-PA, Prourokinase, Urokinase, Streptokinase oder eines ihrer Derivate verwenden. Ein bevorzugtes chromogenes Plasminsubstrat ist Tos-Gly-Pro-Lys-p-Nitroanilin.

Das Verfahren der Erfindung kann bei einer konstanten beliebigen Temperatur zwischen etwa 20 und 40°C durchgeführt werden, dieses wird jedoch vorzugsweise unter physiologischen Bedingungen, also bei 37°C durchgeführt.

Das erfindungsgemäße Verfahren wird zweckmäßigerweise wie folgt durchgeführt:

Bei dem erfindungsgemäßen Bestimmungsverfahren wird vorzugsweise verdünntes PPP verwendet, wobei der Verdünnungsbereich zweckmäßigerweise zwischen 1:5 und 1:25, vorzugsweise jedoch zwischen 1:8 und 1:15, liegt.

Hierbei wird das zu untersuchende plättchenarme Plasma nach an und für sich bekannten Methoden hergestellt, z.B. durch Zentrifugation des zur Untersuchung entnommenen Citrat- oder EDTA-Blutes, zweckmäßigerweise bei 3 000 bis 4 000 x g, vorzugsweise jedoch bei 3 000 bis 3 500 x g und innerhalb von 10 Minuten.

Als Verdünnungsmittel kommen hierbei die bei Aktivitätsmessungen im Plasma üblichen Verdünnungsmittel in Betracht, zweckmäßigerweise isotonische Verdünnungsmittel wie physiologische Kochsalzlösung oder gepufferte Verdünnungsmittel wie Tris-Puffer, NaCl-Puffer oder HEPES-Puffer, z.B. 0,1 M Tris- Puffer pH 7,4, 0,2 M Tris-Puffer pH 7,4, 0,1 M HEPES-Puffer pH 7,4 oder 0,9 % NaCl-Puffer.

Die vorstehend erwähnten Verdünnungsmittel können zusätzlich noch weitere übliche Hilfsstoffe enthalten, z.B. einen Komplexbildner wie 0,02 Gewichtsprozent EDTA, eine oberflächenaktive Substanz wie 0,1 Gewichtsprozent Tween 80 und/oder ein Gewichtsprozent Blutserumalbumin.

Ein erfindungsgemäß bevorzugtes Verdünnungsmittel stellt jedoch eines der vorstehend erwähnten Verdünnungsmittel dar, welches Polyethylenglykol mit einem Molgewicht von 8.000 bis 35.000, vorzugsweise jedoch von 9.000 bis 12.500, und in einer Konzentration von 1 bis 5 Gewichtsprozenten, vorzugsweise jedoch von 2 Gewichtsprozenten, enthält. Durch die erfindungsgemäße Verwendung von Polyethylenglykol wird eine Trübungsverstärkung in der zu messenden Probe erzielt, wobei bei der bevorzugten Verwendung von Polyethylglykol mit einem Molgewicht von 9.000 bis 12.500 gleichzeitig die Gerinnung nicht verzögert und die Gerinnselretraktion nicht zu stark wird.

Selbstverständlich kann einer bereits entsprechend verdünnten Plasmaprobe die erforderliche Menge an Polyethylenglykol mit einem Molgewicht von 8.000 bis 35.000, vorzugsweise jedoch von 9.000 bis 12.500, bis zu einer Konzentration von 1 bis 5 Gewichtsprozenten nachträglich zugefügt werden. Bei der nachträglichen Zugabe wird jedoch bevorzugt Polyethylenglykol mit einem Molgewicht von 9.000 bis 12.500 bis zu einer Konzentration von nur 1,5 Gewichtsprozenten zugegeben.

Das so verdünnte PPP, welches bereits einen Plasminogenaktivator, vorzugsweise rt-PA, in den verschiedenen Verdünnungsschritten entsprechend der gewählten Konzentrationsreihe enthält, wird mit einem thrombinähnlichen Enzym wie Batroxobin zur Gerinnung gebracht. Das klare nichtgeronnene PPP wird während der Gerinnung trübe, wobei die Trübung mit fort schreitender Gerinnung zunimmt. Der Zusatz von Plasminogenaktivatoren vor Beginn der Gerinnung bewirkt, daß das Gerinnsel nach Erreichen eines

3

Gerinnungsmaximus wieder lysiert wird. Die Lyse geht mit einer Abnahme der Trübung einher. Das Lyseende ist durch das Erreichen des Ausgangswertes gekennzeichnet.

Zur Bestimmung der Standardkurve, welche aus plättchenarmem Plasma vor Therapiebeginn erstellt wird, werden beispielsweise 0,1 ml PPP mit 0,9 ml einem Polyethylenglykol enthaltenden entsprechenden Verdünnungsmittel verdünnt und nach Mischen 5 Minuten bei 37°C inkubiert. Anschließend werden beispielsweise 50 $\mu$l Batroxobin zugegeben und nach Mischen die Trübungsänderung photometrisch bei einer Wellenlänge von 340 nm und 37°C aufgezeichnet. Anhand der Standardkurve kann aus dem Zeitintervall bis zum Erreichen von 50 % Lyse die der fibrinolytischen Aktivität im Plasma entsprechende Plasminogenaktivatorkonzentration bestimmt werden. Die Einhaltung einer konstanten Temperatur während einer gesamten Meßreihe ist entscheidend, da die der Lyse zu Grunde liegende enzymatische Reaktion temperaturabhängig ist. Mit abnehmender Temperatur sinkt auch die Geschwindigkeit der Lyse und Werte von Messungen unterschiedlicher Zeitpunkte bei verschiedener Temperatur können nicht mehr in Relation zueinander gesetzt werden.

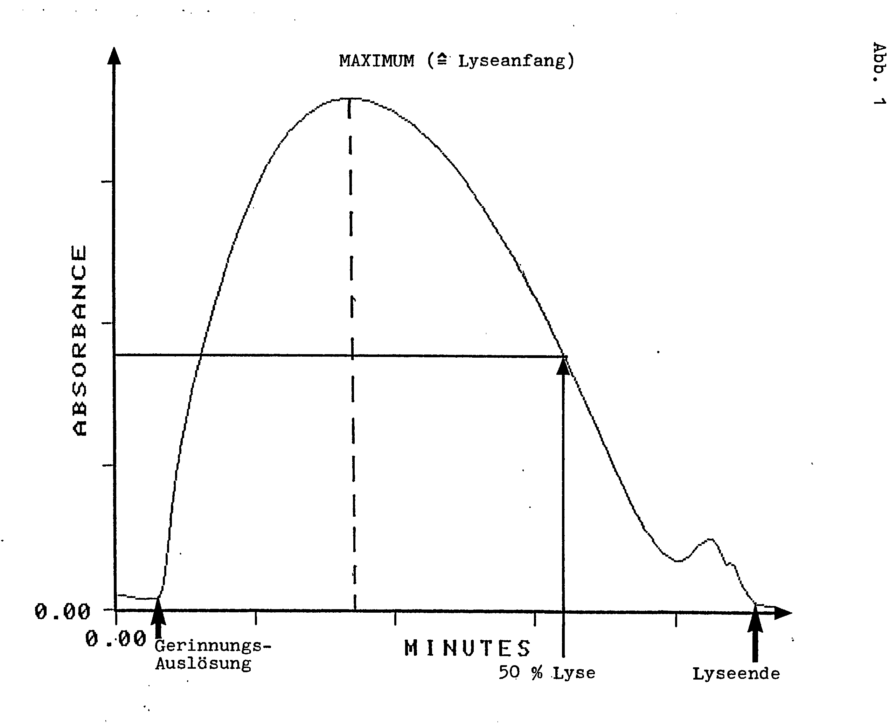

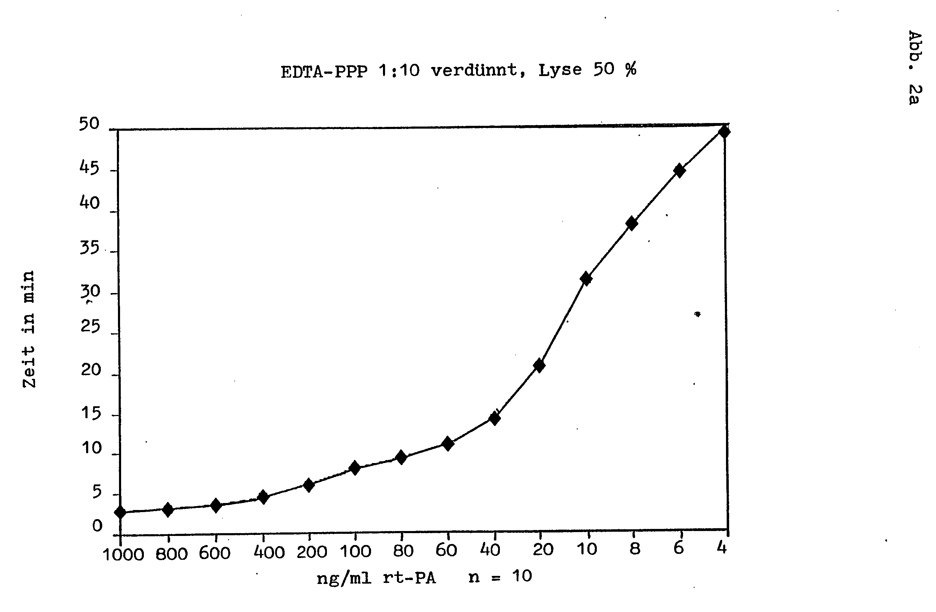

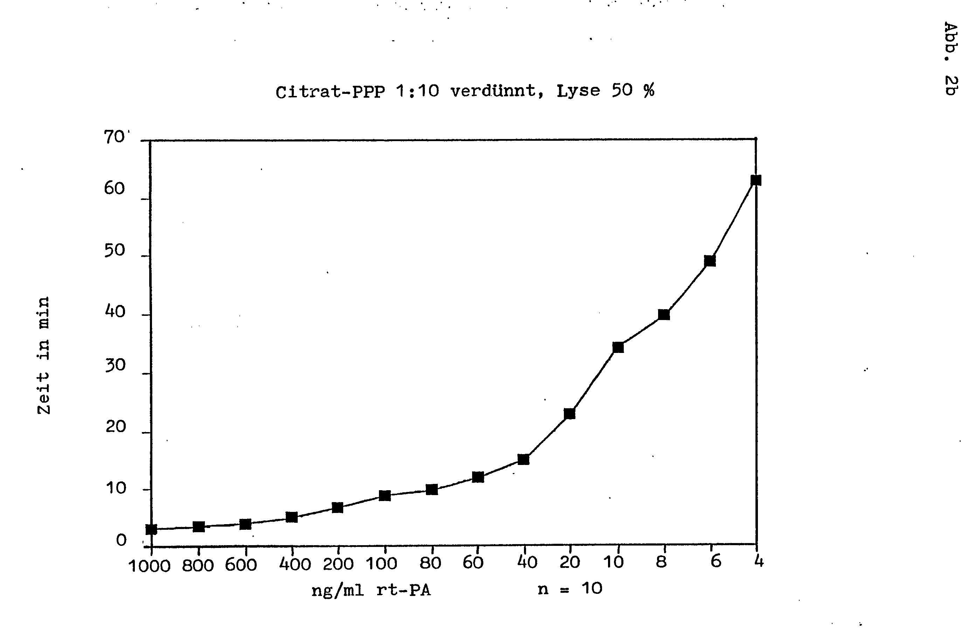

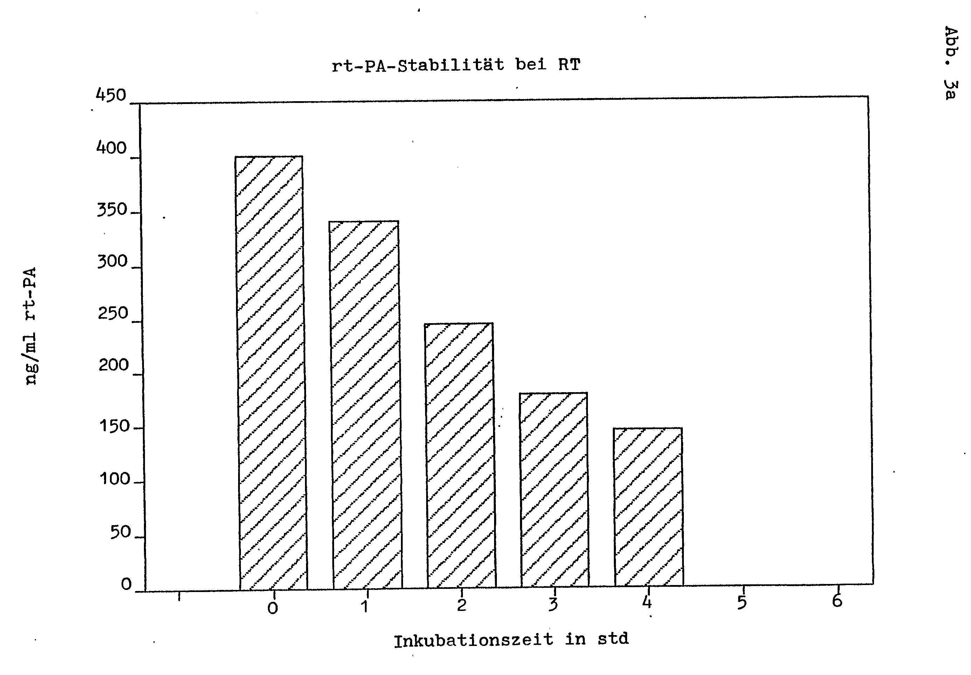

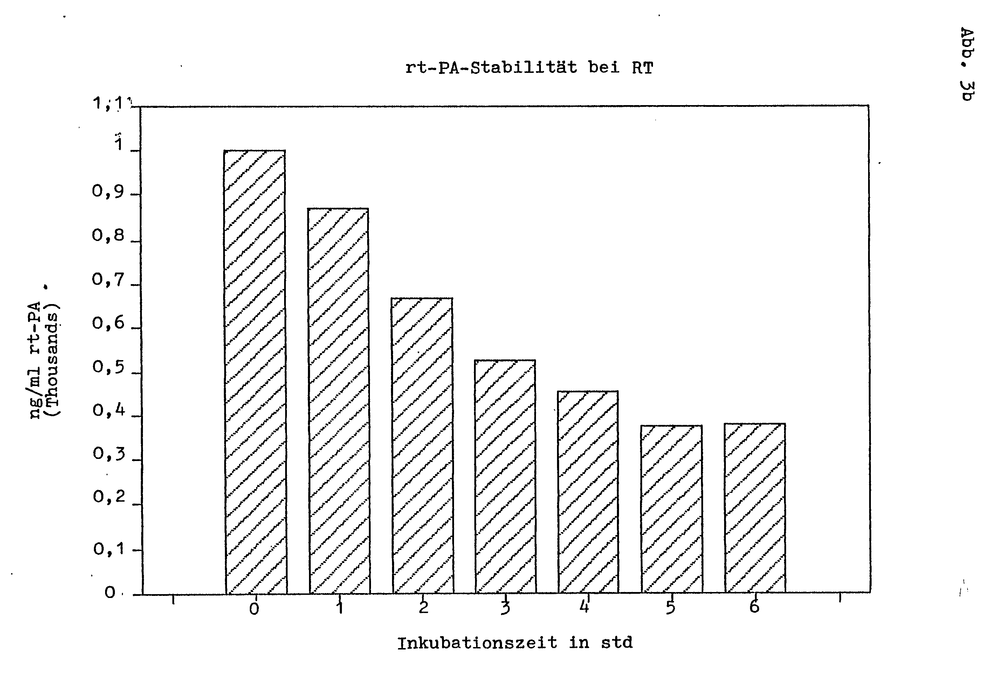

Die Auswertung basiert hierbei auf der Korrelation, daß die Zeit zum Erreichen von 50 % Lyse umgekehrt proportional zur Konzentration des Plasminogenaktivators in der Probe ist. Als Maß für die fibrinolytische Aktivität gilt die Zeit von der Auslösung der Gerinnung bis zum Erreichen einer 50%igen Lyse im Verhältnis zum Gerinnungsmaximum (siehe Abb. 1). Die Werte für die Gesamtaktivität aus Berechnungen, basierend auf dem Erreichen von 50 % Lyse, korrelieren sehr gut mit Werten, welche bei Zugrundelegen des Lyseendes erhalten wurden. Daher ist es sinnvoll, den Test bereits nach Erreichen von 50 % Lyse zu beenden, da hierdurch der Zeitaufwand für jede Ein zelmessung und die Gesamtdurchführung der Methode deutlich reduziert wird.

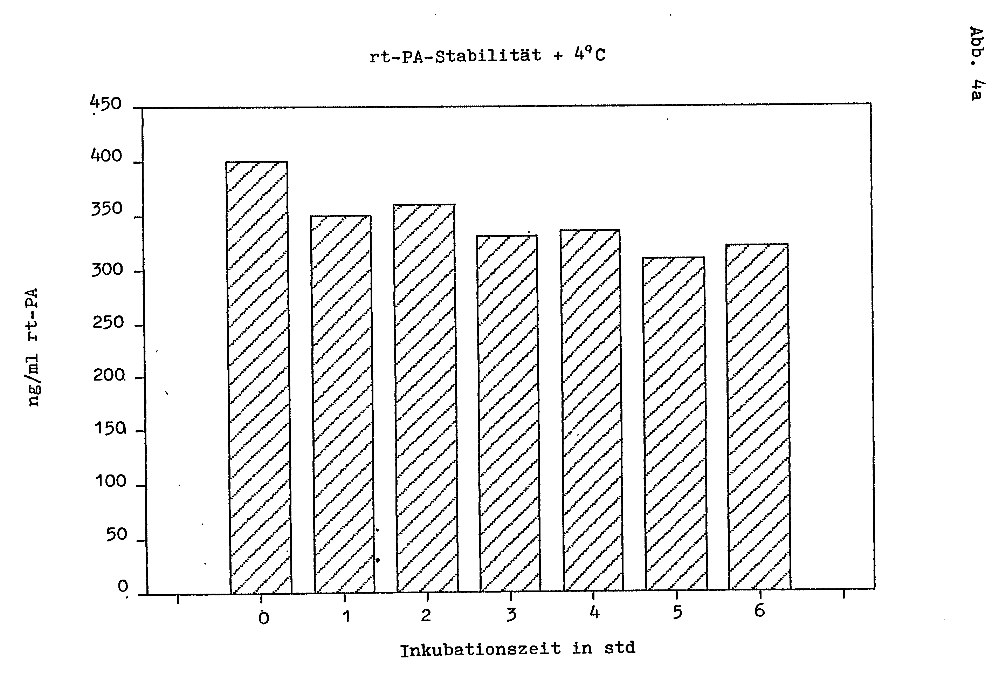

Beispiele für Standardkurven der fibrinolytischen Aktivität (50 % Lyse) von rt-PA werden in Abb. 2 gezeigt.

Abb. 2a zeigt eine Standardkurve in 1:10 verdünntem PPP aus EDTA-Blut.

Abb. 2b zeigt eine Standardkurve in 1:10 verdünntem PPP aus Citrat-Blut.

Es ist zu erkennen, daß die Standardkurven über den gesamten Meßbereich von 2 bis 1000 ng/ml nicht linear sind. Bei niederen Dosierungen von rt-PA, wie z.B. im Indikationsbereich "tiefe Venenthrombose" sind jedoch Plasmaspiegel nur im Bereich von 2 bis 40 ng/ml zu erwarten. Der Verlauf der Kurve in diesem engen Bereich darf als linear angenommen werden.

Eine besonders bevorzugte Ausführungsform des gesamten Testes gliedert sich daher in folgende methodische Einzelschritte:

a) Abnahme von Citrat- oder EDTA-Blut vor Beginn der fibrinolytischen Therapie zur Erstellung einer Standardkurve.

b) Gewinnung von plättchenarmem Plasma (platelet poor plasma = PPP) und 1:10 Verdünnung des PPP mit einem entsprechenden Verdünnungsmittel.

c) Erstellen einer Standardkurve für dieses verdünnte PPP durch Zugabe des Plasminogenaktivators im Konzentrationsbereich von 2 bis 1000 ng/ml, vorzugsweise von 2 bis 100 ng/ml.

d) Wiederholung von a) und b) während der fibrinolytischen Therapie.

e) Bestimmung der aktuellen fibrinolytischen Gesamtaktivität im Plasma des Patienten anhand der Standardkurve.

Anhand der erhaltenen Ergebnisse erfolgt dann eine eventuell erforderliche Korrektur der infundierten Plasminogenaktivatormenge zur Einstellung der gewünschten fibrinolytischen Gesamtaktivität im Blut des Patienten.

Die Einzelschritte werden hierbei vorzugsweise wie folgt durchgeführt:

a) Die Blutabnahme erfolgt unter den üblichen Bedingungen. EDTA-Blut: 1 Teil 2 % EDTA + 9 Teile Blut.

Citrat-Blut: 1 Teil 3.8 % Natrium-Citrat + 9 Teile Blut.

b) Das Blut wird sofort nach Abnahme 15 Minuten bei 3300 x g und 20°C zentrifugiert. Nach der Zentrifugation wird das überstehende PPP abgehoben und 1:10 verdünnt. Verdünnungsmittel für PPP aus EDTA-Blut:

2 % Polyethylenglykol 10.000 mit einem Molgewichtsbereich von 9.000 bis 12.500 und 0,1 % Tween 80 in physiologischer Kochsalz-Lösung.

Verdünnungsmittel für PPP aus Citratblut:

2 % Polyethylenglykol 10.000 mit einem Molgewichtsbereich von 9.000 bis 12.500, 0.1 % Tween 80 und 0.02 % EDTA in physiologischer NaCl-Lösung.

c) Als Beispiel für den Plasminogenaktivator wurde rt-PA (Actilyse) verwendet, wobei zur Verdünnung von rt-PA folgender Puffer verwendet wurde: 0.5 M L-Arginin, 0.1 % Tween 80 und 0.05 M $Na_2HPO_4$, pH 7.4.

4

Zur Erstellung der Standardkurve werden die Lyseaktivitäten im Konzentrationsbereich von 2 bis 1000 ng/ml rt-PA in PPP aus EDTA- oder Citrat-Blut mit den Zusammensetzungen des Einzeltests gemäß nachfolgendem Schema bestimmt (rt-PA Endkon zentration in PPP zur Erstellung der Standardkurve):

1000 ng/ml, 800 ng/ml, 600 ng/ml, 400 ng/ml, 200 ng/ml, 100 ng/ml, 80 ng/ml, 60 ng/ml, 40 ng/ml, 20 ng/ml, 10 ng/ml, 8 ng/ml, 6 ng/ml, 4 ng/ml und 2 ng/ml

Bei Bedarf kann der Konzentrationsbereich enger oder weiter gewählt werden oder weniger Meßpunkte bestimmt werden.

d) und e) Zur Bestimmung des fibrinolytischen Status eines Patienten unter Therapie werden 0.1 ml PPP, gewonnen vom Blut des Patienten, mit 0.9 ml einem der vorstehend erwähnten geeigneten Verdünnungsmittel verdünnt und nach Mischen 5 Minuten bei 37° C inkubiert. Anschließend werden 50 $\mu$l Batroxobin zugegeben und nach Mischen die Trübungsänderung photometrisch bei einer Wellenlänge von 340 nm aufgezeichnet. Anhand der Standardkurve kann aus dem Zeitintervall bis zum Erreichen von 50 % Lyse die der fibrinolytischen Aktivität im Plasma entsprechende Plasminogenaktivatorkonzentration bestimmt werden.

Entspricht die fibrinolytische Aktivität nicht dem Sollwert, so kann diese durch Anpassung der Infusionsrate des Plasminogenaktivators entsprechend korrigiert werden.

Ein weiterer Vorteil der erfindungsgemäßen Methode liegt darin, daß diese auch geeignet ist, niedrige Plasminogenaktivatorkonzentrationen in Gegenwart von Heparin zu bestimmen. Dies ist für eine fibrinolytische Therapie von großer Bedeutung, da diese im Normalfall auch unter Einsatz von Heparin durchgeführt wird.

Zum Beleg dieses Vorteils wurde der Einfluß von Heparin auf die Lysezeit wie folgt geprüft:

Bei der Durchführung des erfindungsgemäßen Testes wurde dem Citrat- bzw. EDTA-Plasma Heparin entsprechend nach folgendem Schema zugesetzt:

| a) Citrat-PPP | | | | | |
|---|---|---|---|---|---|
| 8 ng/ml rt-PA | Citrat - PPP | | | | |
| Heparin I.U./ml | 0 | 0.1 | 1 | 5 | 10 |
| Lyse 50 % in min. | | | | | |
| 1 | 28,67 | 31,30 | 31,00 | 30,67 | 39,33 |
| 2 | 27,33 | 28,67 | 30,67 | 31,67 | 31,67 |
| Mittelwert | 28,00 | 29,99 | 30,94 | 31,17 | 35,50 |

| b) EDTA-PPP | | | | | |
|---|---|---|---|---|---|
| 8 ng/ml rt-PA | EDTA - PPP | | | | |
| Heparin I.U./ml | 0 | 0.1 | 1 | 5 | 10 |
| Lyse 50 % in min. | | | | | |
| 1 | 27,00 | 26,00 | 26,67 | 29,00 | 31,00 |
| 2 | 24,00 | 23,33 | 25,67 | 25,67 | 25,67 |
| Mittelwert | 25,50 | 26,67 | 26,17 | 27,34 | 28,33 |

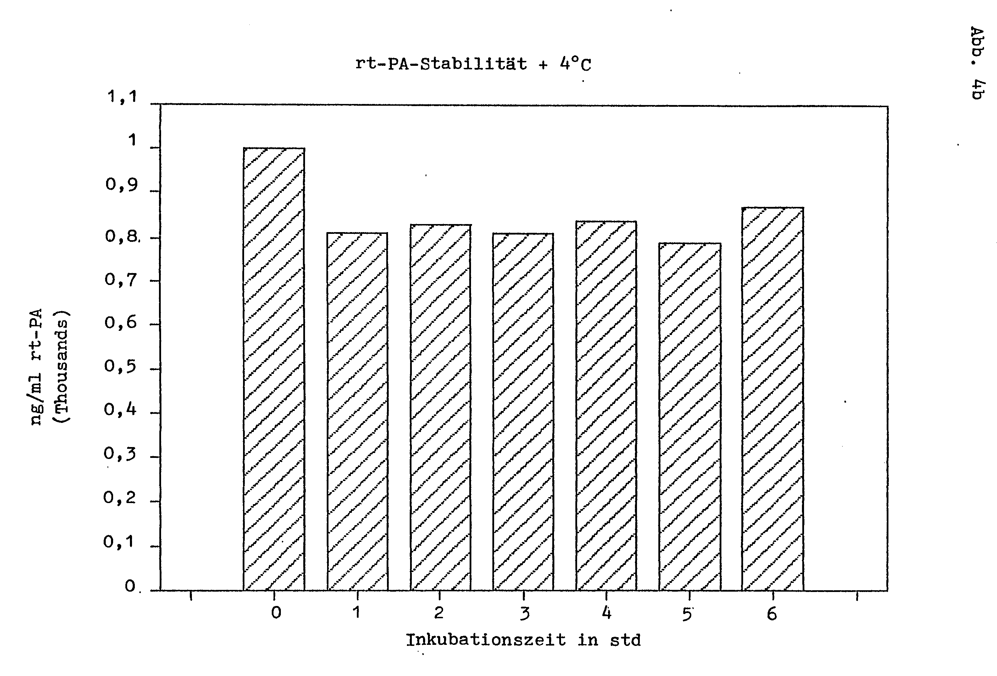

Kann die Probe nicht unmittelbar nach der Blutentnahme aufbereitet und gemessen werden, so sollte das Plasma bei 4° C aufbewahrt werden, da bei Raumtemperatur (RT) die meßbare fibrinolytische Aktivität schnell abnimmt (Abb. 3a und 3b).

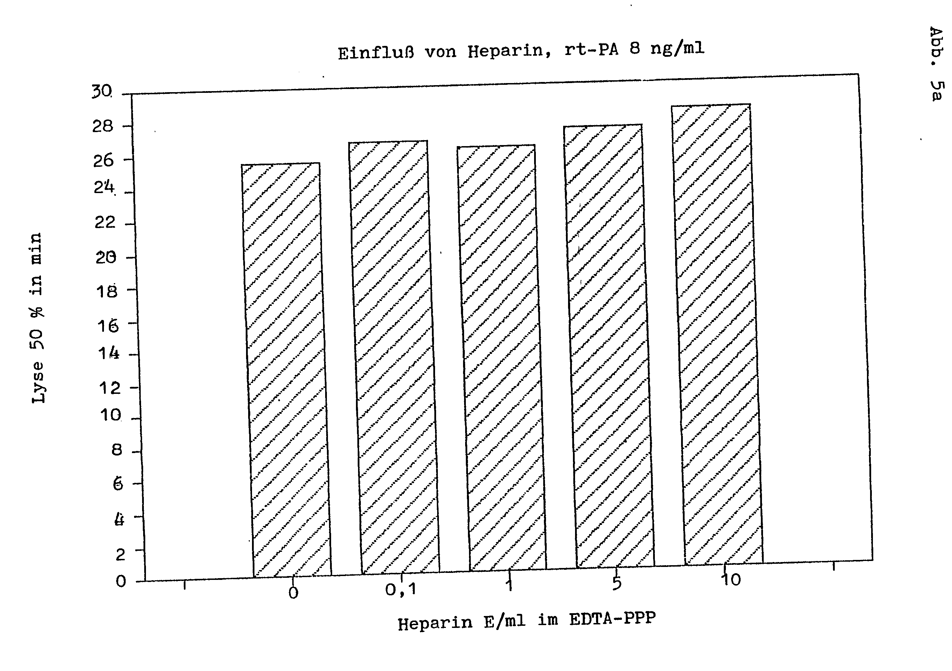

Bei 4°C beschränkt sich dieser Verlust auf ca. 20 % innerhalb einer Stunde und nimmt in den folgenden 5 Stunden nicht weiter zu (Abb. 4a und 4b), so daß die erhaltenen Meßwerte um diesen Verlust korrigiert werden können. Messungen zu einem späteren Zeitpunkt sind nicht sinnvoll, da diese keine Korrektur der Infusion zur Einstellung der fibrinolytischen Aktivität innerhalb eines adäquaten Zeitraumes erlauben.

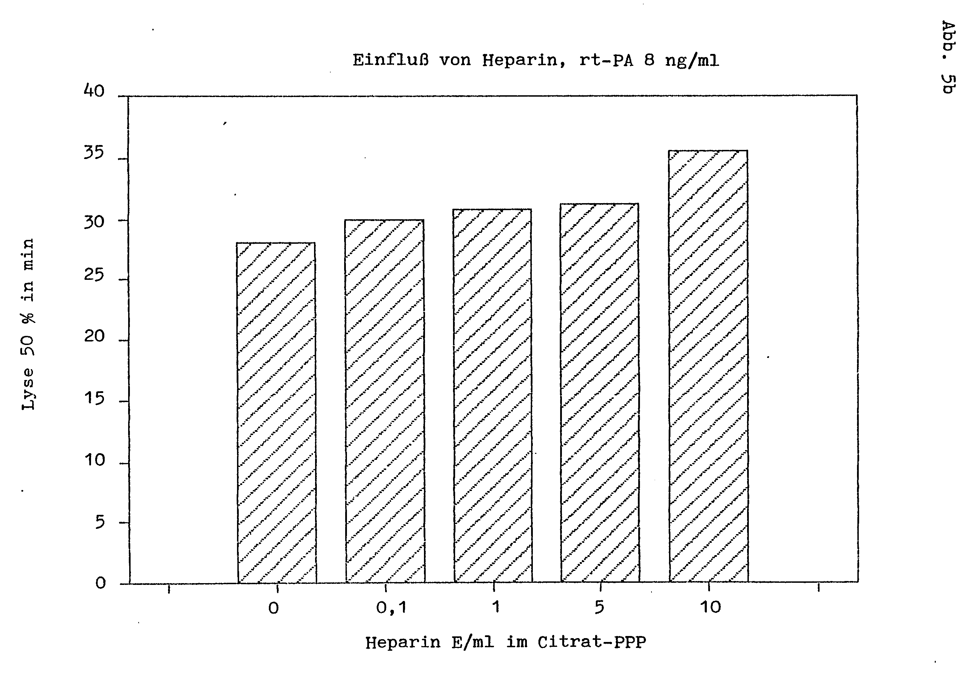

Ferner geht aus Abb. 5a und 5 hervor, daß selbst bei sehr niedriger eingesetzter rt-PA Konzentrationen der Einfluß von Heparinzusatz bis zu 1 I.U./ml relativ gering ist, das heißt, die Zeit bis zu 50 % Lyse oder dem Lyseende wird nicht wesentlich verlängert oder verkürzt (≤ 10 %).

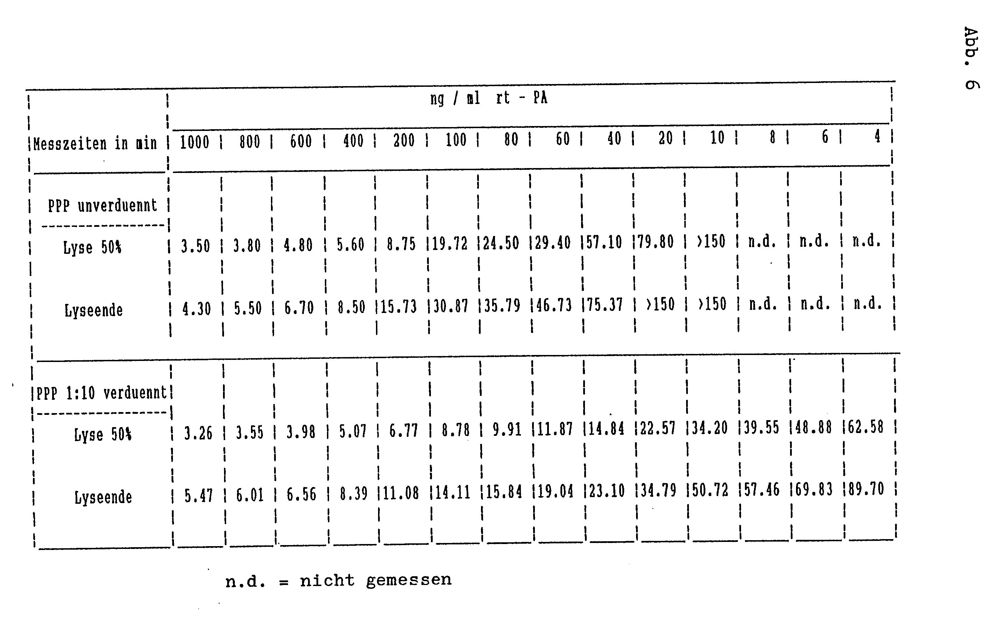

Die Überlegenheit der erfindungsgemäßen Bestimmungsmethode ist aus Abb. 6 ersichtlich:

Proben, die in unverdünntem Zustand sehr lange Lysezeiten (Lyse 50 % oder Lyseende) aufweisen und daher gemäß Verfahren der DE-A-3.502.878 nicht mehr gemessen werden können (Lyse 50% bei > 3 Stunden), sind gemäß den erfindungsgemäßen Verfahren ohne weiteres bestimmbar, da die erforderlichen Meßzeiten, insbesondere bei niederen Plasminogenaktivatorkonzentrationen, erheblich verkürzt werden konnten.

Dies ist bei Messungen während einer Therapie, die unter Anwendung von niederen Plasminogenaktivatorkonzentrationen durchgeführt werden, z.B. bei der Therapie von tiefen Venenthrombosen, Lungenembolien usw., von großer Bedeutung.

Außerdem tritt bei der Durchführung des erfindungsgemäßen Verfahrens unter Verwendung von physiologischer Kochsalzlösung als Verdünnungsmittel und unter Zusatz von 2 Gewichtsprozenten käuflichem Polyethylenglykol 10.000, welches ein mittleres Molgewicht von 9.000 bis 12.500 aufweist, keine unerwünschte Retraktion in der Probe auf.

Das nachstehende Beispiel erläutert die Erfindung weiter in Verbindung mit der vorstehenden Beschreibung und den beigefügten Abbildungen:

## Beispiel

### Reagenzien:

Reptilase 20 PU/ml
rt-PA (2 bis 1000 ng/ml nur für Standard)
Verdünnungspuffer für Citrat-PPP:
0,9%ige Natriumchloridlösung
2,0 % Polyethylenglykol 10.000
0,1 % Tween 80
0,02 % EDTA
Verdünnungspuffer für EDTA-PPP:
0,9%ige Natriumchloridlösung
2,0 % Polyethylenglykol 10.000
0,1 % Tween 80

### Testansatz:

10 µl rt-PA (2 bis 1000 ng nur für Standard)
100 µl PPP
300 µl Verdünnungsmittel (0,9 % Natriumchloridlösung + 2,0 % Polyethylenglykol 10.000)
Die Probe wird gut durchmischt, 5 Minuten lang bei 37°C inkubiert, dann mit 50 µl Reptilase versetzt und anschließend erneut gut durchmischt.

Nach der Gerinnungsauslösung wird sofort die Extinktionszunahme und -abnahme photometrisch bei einer Wellenlänge bei 340 nm und einer Meßtemperatur von 37°C verfolgt.

### Erstellen einer rt-PA-Dosiswirkungskurve im 1:10 und 1:20 verdünnten Citrat- und EDTA-PPP

### Reagenzien:

6

Reptilase (Boehringer Mannheim, Bestellnummer 126 560)
Inhalt eines Vials in 1 ml aqua dest. lösen
Citratblut: 1 Teil 3,8 % Na-Citrat + 9 Teile Blut
EDTA-Blut: 1 Teil 2 % EDTA + 9 Teile Blut
rt-PA-Standard: 1 mg/ml
0,5 M Argininpuffer pH 7.4:
0,5 M L-Arginin +
0,1 % Tween 80 +
0,05 M Dinatriumhydrogenphosphat
Verdünnungspuffer für Citratblut:
physiologische NaCl-Lösung +
2 % Polyethylenglycol 10.000 +
0,1 % Tween 80 +
0,02 % EDTA
Verdünnungspuffer für EDTA-Blut:
physiologische NaCl-Lösung +
2 % Polyethylenglycol 10.000 +
0,1 % Tween 80

Durchführung:

Plasmagewinnung:

Die Blutabnahme erfolgt nach den üblichen Bedingungen an der Vena cubitalis. Das Blut wird sofort nach der Abnahme 15 Minuten bei 3300 x g (4000 U/min.) bei 20°C zentrifugiert. PPP abheben und weiterverarbeiten.

Herstellung des rt-PA-Standards (siehe Abb. 1):

Es werden die Lyseaktivitäten folgender rt-PA-Konzentrationen im Citrat-PPP bzw. EDT-PPP bestimmt 1000, 800, 600, 400, 200, 100, 80, 60, 40, 20, 8, 6, 4 und 2 ng/ml. Die rt-PA-Verdünnungen werden nach dem folgenden Schema in Argininpuffer hergestellt:

| Menge rt-PA-Lösung | Konz.der rt-PA-Lösung | + 0,5 M Argininpuffer | = Konz.der rt-PA-Lösung | zugegeben zu 1 ml PPP | rt-PA-Konz. in PPP |
|---|---|---|---|---|---|
| 50 μl | 1 mg/ml | 450 μl | 100 μg/ml | 10 μl | 1000 ng/ml |
| 40 μl | 1 mg/ml | 460 μl | 80 μg/ml | 10 μl | 800 ng/ml |
| 30 μl | 1 mg/ml | 470 μl | 60 μg/ml | 10 μl | 600 ng/ml |
| 20 μl | 1 mg/ml | 480 μl | 40 μg/ml | 10 μl | 400 ng/ml |
| 10 μl | 1 mg/ml | 490 μl | 20 μg/ml | 10 μl | 200 ng/ml |
| 50 μl | 100 μg/ml | 450 μl | 10 μg/ml | 10 μl | 100 ng/ml |
| 50 μl | 80 μg/ml | 450 μl | 8 μg/ml | 10 μl | 80 ng/ml |
| 50 μl | 60 μg/ml | 450 μl | 6 μg/ml | 10 μl | 60 ng/ml |
| 50 μl | 40 μg/ml | 450 μl | 4 μg/ml | 10 μl | 40 ng/ml |
| 50 μl | 20 μg/ml | 450 μl | 2 μg/ml | 10 μl | 20 ng/ml |
| 50 μl | 10 μg/ml | 450 μl | 1 μg/ml | 10 μl | 10 ng/ml |
| 50 μl | 8 μg/ml | 450 μl | 0.8 μg/ml | 10 μl | 8 ng/ml |
| 50 μl | 6 μg/ml | 450 μl | 0.6 μg/ml | 10 μl | 6 ng/ml |
| 50 μl | 4 μg/ml | 450 μl | 0.4 μg/ml | 10 μl | 4 ng/ml |
| 50 μl | 2 μg/ml | 450 μl | 0.2 μg/ml | 10 μl | 2 ng/ml |

Kit zur Bestimmung der fibrinolytischen Eigenschaft im Human-Plasma

a) Für 3 x 20 Bestimmungen im Citrat-Plasma (1 Teil 3.8 % Na-Citrat + 9 Teile Blut):

Reagenzien:

60 ml Verdünnungspuffer:
0,9 % NaCl-Lösung +
2 % Polyethylenglycol 10.000 +
0,1 % Tween 80 +
0,02 % EDTA
3 x 20 PU/Vial Reptilase (Inhalt eines Vials in 1 ml aqua dest. lösen)
rt-PA-Standard: 1 mg pro ml
50 ml Argininpuffer (zur Verdünnung des rt-PA-Standards):
0,5 M L-Arginin pH 7.4 +
0,1 % Tween 80 +
0,05 M Dinatriumhydrogenphosphat
b) Für 3 x 20 Bestimmungen im EDTA-Plasma (1 Teil 2 % EDTA + 9 Teile Blut):

Reagenzien:

60 ml Verdünnungspuffer:
0,9 % NaCl-Lösung +
2 % Polyethylenglycol 10.000 +
0,1 % Tween 80
3 x 20 PU/Vial Reptilase (Inhalt eines Vials in 1 ml aqua dest. lösen)
rt-PA-Standard: 1 mg pro ml
50 ml Argininpuffer (zur Verdünnung des rt-PA-Standards):
0,5 M L-Arginin pH 7.4 +
0,1 % Tween 80 +
0,05 M Dinatriumhydrogenphosphat

**Ansprüche**

1. Verfahren zur Bestimmung der fibrinolytischen Gesamtaktivität im Plasma, dadurch gekennzeichnet, daß man einer verdünnten plättchenarmen Plasmaprobe

a) eine zur Trübungsbildung ausreichende Menge an Fibrin zusetzt oder diese in situ erzeugt und die zeitabhängige Trübungsänderung oder die zeitabhängige Bildung von Fibrinspaltprodukten mißt oder

b) ein chromogenes Plasminsubstrat und eine nicht zur Trübungsbildung ausreichende Menge an Fibrin, Fibrinogenspaltprodukten oder eines Fibrin in situ erzeugenden Enzymes zusetzt und die zeitabhängige Farbbildung mißt.

2. Verfahren nach Anspruch 1, dadurch gekennzeichnet, daß man in einer verdünnten Plasmaprobe Fibrin in situ durch Zusatz von einem thrombinähnlichen Enzym wie Batroxobin erzeugt und die zeitabhängige Farbbildung mißt.

3. Verfahren nach Anspruch 1 oder 2, dadurch gekennzeichnet, daß man der verdünnten plättchenarmen Plasmaprobe Polyethylenglykol mit einem Molgewicht von 8.000 bis 35.000, vorzugsweise von 9.000 bis 12.500, bis zu einer Endkonzentration von 1 bis 5 Gewichtsprozenten, vorzugsweise von 2 Gewichtsprozenten, zusetzt.

4. Verfahren nach mindestens einem der Ansprüche 1 bis 3, dadurch gekennzeichnet, daß das verwendete Verdünnungsmittel zusätzlich einem Komplexbildner wie 0,02 Gewichtsprozent EDTA und/oder eine oberflächenaktive Substanz wie 0,1 Gewichtsprozent Tween 80 enthält.

5. Verfahren nach Anspruch 1 oder 2, dadurch gekennzeichnet, daß die Auswertung nach Erreichen von 50 % Lyse erfolgt.

6. Verwendung von verdünntem plättchenarmem Plasma bei der Bestimmung der fibrinolytischen Gesamtaktivität im Plasma.

7. Verwendung nach Anspruch 6, dadurch gekennzeichnet, daß man der verdünnten plättchenarmen Plasmaprobe Polyethylenglykol mit einem Molgewicht von 8.000 bis 35.000, vorzugsweise von 9.000 bis 12.500, bis zu einer Endkonzentration von 1 bis 5 Gewichtsprozenten, vorzugsweise von 2 Gewichtsprozenten, zusetzt.

8. Verwendung nach Anspruch 6 oder 7, dadurch gekennzeichnet, daß das Verdünnungsmittel zusätzlich einen Komplexbildner wie 0,02 Gewichtsprozent EDTA und/oder eine oberflächenaktive Substanz wie 0,1 Gewichtsprozent Tween 80 enthält.

9. Verdünntes plättchenarmes Plasma, dadurch gekennzeichnet, daß dieses Polyethylenglykol mit einem Molgewicht von 8.000 bis 35.000, vorzugsweise von 9.000 bis 12.500, und in einer K?nzentration von 1 bis 5 Gewichtsprozenten, vorzugsweise von 2 Gewichtsprozenten, enthält.

10. Kit, der zur Bestimmunng der fibrinolytischen Gesamtaktivität in verdünntem plättchenarmen Plasma gemäß dem Verfahren der Ansprüche 1 bis 3 geeignet ist, dadurch gekennzeichnet, daß dieser aus

a) einer vorbestimmten Menge von rt-PA, vorzugsweise in lyophilisierter Form,

b) einer vorbestimmten Menge eines thrombinähnlichen Enzyms,

c) einem zur Verdünnung von rt-PA geeigneten Puffer und

d) einem zur Verdünnung von Plasma geeigneten Puffer, welcher Polyethylenglykol mit einem durchschnittlichen Molgewicht von 8.000 bis 35.000 enthält, vorzugsweise jedoch von 9.000 bis 12.500, besteht.

Abb. 1

EP 0 378 139 A2

MAXIMUM (≙ Lyseanfang)

ABSORBANCE

0.00

0.00    Gerinnungs-
              Auslösung

MINUTES

50 % Lyse

Lyseende

Abb. 2a

EP 0 378 139 A2

EDTA-PPP 1:10 verdünnt, Lyse 50 %

ng/ml rt-PA    n = 10

Citrat-PPP 1:10 verdünnt, Lyse 50 %

Abb. 2b

EP 0 378 139 A2

rt-PA-Stabilität bei RT

Abb. 3a

EP 0 378 139 A2

rt-PA-Stabilität bei RT

Inkubationszeit in std

ng/ml rt-PA (Thousands)

Abb. 3b

EP 0 378 139 A2

rt-PA-Stabilität + 4°C

Abb. 4a

EP 0 378 139 A2

Abb. 4b

rt-PA-Stabilität + 4°C

ng/ml rt-PA
(Thousands)

Inkubationszeit in std

Abb. 5a

Einfluß von Heparin, rt-PA 8 ng/ml

Lyse 50 % in min

Heparin E/ml im EDTA-PPP

Abb. 5b

Einfluß von Heparin, rt-PA 8 ng/ml

Heparin E/ml im Citrat-PPP

Lyse 50 % in min

Abb. 6

EP 0 378 139 A2

| Messzeiten in min | ng / ml rt - PA | | | | | | | | | | | | | |
|---|---|---|---|---|---|---|---|---|---|---|---|---|---|---|
| | 1000 | 800 | 600 | 400 | 200 | 100 | 80 | 60 | 40 | 20 | 10 | 8 | 6 | 4 |
| PPP unverduennt | | | | | | | | | | | | | | |
| Lyse 50% | 3.50 | 3.80 | 4.80 | 5.60 | 8.75 | 19.72 | 24.50 | 29.40 | 57.10 | 79.80 | >150 | n.d. | n.d. | n.d. |
| Lyseende | 4.30 | 5.50 | 6.70 | 8.50 | 15.73 | 30.87 | 35.79 | 46.73 | 75.37 | >150 | >150 | n.d. | n.d. | n.d. |
| PPP 1:10 verduennt | | | | | | | | | | | | | | |
| Lyse 50% | 3.26 | 3.55 | 3.98 | 5.07 | 6.77 | 8.78 | 9.91 | 11.87 | 14.84 | 22.57 | 34.20 | 39.55 | 48.88 | 62.58 |
| Lyseende | 5.47 | 6.01 | 6.56 | 8.39 | 11.08 | 14.11 | 15.84 | 19.04 | 23.10 | 34.79 | 50.72 | 57.46 | 69.83 | 89.70 |

n.d. = nicht gemessen